# EUROPÄISCHE PATENTSCHRIFT

(11) **EP 0 771 546 B1**
(45) Veröffentlichungstag und Bekanntmachung des Hinweises auf die Patenterteilung: **11.09.2002**
(21) Anmeldenummer: 96117608.8
(22) Anmeldetag: 04.11.1996
(51) Int. Cl.: A61B 5/00, A61B 5/026

(54) **Auswerteverfahren zur Detektion des Blutflusses und/oder intra- und/oder extrakorporal fliessender Flüssigkeiten in biologischem Gewebe**
Method for detection of blood flow and/or intra- and/or extracorporal flowing liquid in a biological tissue
Procédé pour la détection du flux sanguin et/ou d'un liquide intra- et/ou extracorporel coulant dans un tissue biologique

(30) Priorität: 03.11.1995 DE 19541043; 26.02.1996 EP 96102821; 29.07.1996 DE 19630381
(43) Veröffentlichungstag der Anmeldung: 07.05.1997
(73) Patentinhaber: Ott, Lutz, 35463 Fernwald (DE)
(72) Erfinder: Ott, Lutz, 35463 Fernwald (DE)
(74) Vertreter: Müller, Eckhard, Dr.

(56) Entgegenhaltungen:
- EP-A- 0 728 440
- DE-A- 4 314 835
- US-A- 4 590 948
- US-A- 4 596 254
- AIZU Y ET AL: "BIO-SPECKLE PHENOMENA AND THEIR APPLICATION TO THE EVALUATION OF BLOOD FLOW" OPTICS AND LASER TECHNOLOGY, Bd. 23, Nr. 4, 1. August 1991, Seiten 205-219, XP000262901
- DOUGHERTY G: "A LASER DOPPLER FLOWMETER USING VARIABLE COHERENCE TO EFFECT DEPTH DISCRIMINATION" REVIEW OF SCIENTIFIC INSTRUMENTS, Bd. 63, Nr. 5, 1. Mai 1992, Seiten 3220-3221, XP000301265

## Beschreibung

Die Erfindung betrifft ein Auswerteverfahren zur Detektion des Blutflusses und/oder intra- und/oder extrakorporal fließender Flüssigkeiten in menschlichem, tierischem oder biologischem Gewebe.

Ein Verfahren mit den eingangs genannten Merkmalen ist aus der US 4 590 948 bekannt und dient zur Messung der Oberflächendurchblutung. Dazu werden die aus dem Gewebe austretenden Photonen in zwei Flächenbereichen erfaßt, die äquidistant zum Eintrittsbereich angeordnet sind. Die Frequenz und Intensität der Photonen wird mittels Detektoren erfaßt. Eine tiefenselektive Bestimmung der Konzentration von Glukose ist mit dieser bekannten Vorrichtung nicht möglich.

Die DE 43 14 835 A1 offenbart eine Vorrichtung und ein Verfahren zur Bestimmung der Konzentration von Glukose in einer biologischen Matrix. Dabei sind mindestens zwei Detektionsmessungen vorgesehen, bei denen jeweils Licht als Primärlicht in die biologische Matrix eingestrahlt wird, das in der biologischen Matrix entlang einem Lichtweg propagiert, und eine als Sekundärlicht austretende Lichtintensität gemessen wird. Weiterhin ist eine Auswertung vorgesehen, bei der aus den Intensitätsmeßwerten der Detektionsmessung mittels eines Auswertealgorithmus und einer Kalibration die Glukosekonzentration abgeleitet wird. Auch bei diesem Verfahren ist eine tiefenselektive Flußdetektion nicht möglich.

Aus der nicht vorveröffentlichten EP 0 728 440 A2 sind ein Auswerteverfahren und eine Vorrichtung zur tiefenselektiven, nichtinvasiven Detektion von Muskelaktivitäten bekannt, wobei man Photonen einer kohärenten, monochromatischen Lichtquelle in das Gewebe durch einen ersten Bereich eintreten läßt, die in unterschiedlichen Abständen von diesem ersten Bereich aus dem Gewebe wieder austretenden Photonen bzgl. Frequenz und Anzahl bzw. Intensität detektiert und aus den Informationen Frequenz und/oder Anzahl bzw. Intensität und/oder Austrittsort der wieder austretenden Photonen mittels eines Auswerteprogrammes und/oder -algorithmus tiefenselektive Rückschlüsse auf die Stärke der Muskelaktivitäten und/oder Anzahl der aktiven Muskeln und/oder räumliche Lage der aktiven Muskeln im Gewebe gewinnt.

Die Haut als Grenzorgan zwischen Mensch und Umwelt erfüllt vielfältige Funktionen. So dient sie zur Erfüllung regulatorischer wie immunologischer Aufgaben und nicht zuletzt auch als Sinnesorgan. Die Haut setzt sich grob aus der Epidermis und der darunter liegenden Schicht, dem Korium bestehend aus Nerven, Muskeln und Kapillargefäßen, zusammen. Durch das Zusammenspiel dieser Muskeln, Nerven und Kapillargefäße erfolgt die Regulation der Mikrozirkulation und die Kontrolle aller anderen, der Haut obliegenden Aufgaben. Eine Bestimmung und Kontrolle der Hautdurchblutung kann nun dazu dienen, Schwankungen oder Störungen derselben festzustellen und eine medizinische Diagnose zu unterstützen. Nachfolgend ist nur ein Auszug der vielfältigen Anwendungsbeispiele stichwortartig aufgelistet:
- Diagnose von Hauterkrankungen, z. B. Sclerodermia, Psoriasis
- Lokalisierung von Arterieskleroseerscheinungen
- Hilfestellung bei der Erkennung diabetischer Mikroangiophatie
- Beobachtung der arteriellen Vasomotion
- Überwachung der Sympathikusfunktionen in der Regionalanästhesie
- Durchblutungskontrolle bei Transplantationen, etc.

Seit Jahrzehnten werden Laser-Doppler-Systeme zur Hautgefäßdiagnostik eingesetzt. Die größte Einschränkung der herkömmlichen Laser-Doppler-Systeme beruht auf deren geringen effektiven Eindringtiefe und der Analyse "richtungsunabhängiger" Signale. Dadurch reduziert sich der Einsatz auf die Messung der Mikrozirkulation der Haut, wobei ein Meßvolumen von der Größe einer Halbkugel mit einem Durchmesser von max. 1 mm untersucht werden kann. Außerdem liefern die bisher zum klinischen Einsatz gekommenen Geräte für die Durchblutung der Haut aus methodischen und meßtechnischen Gründen keine miteinander vergleichbaren Meßwerte. Die Anwender dieser herkömmlichen Systeme, z. B. Flowmeter, sind dazu übergegangen, vorwiegend das Zeitverhalten der Meßsignale nach definierten Stimulationen zur Beurteilung der Mikrozirkulation heranzuziehen.

Der Erfindung liegt daher die Aufgabe zugrunde, ein Auswerteverfahren zur Detektion des Blutflusses und/oder intra- und/oder extrakorporal fließender Flüssigkeiten in biologischem Gewebe anzugeben.

Das erfindungsgemäße Auswerteverfahren ist im wesentlichen dadurch gekennzeichnet, daß die Flächenbereiche in unterschiedlichen Abständen von dem ersten Bereich angeordnet sind und man aus detektierten Interferenzmustern oder Frequenzschwebungen in Verbindung mit dem jeweiligen Flächenbereich als Austrittsort der wieder austretenden Photonen mittels eines Auswerteprogramms oder -algorithmus tiefenselektive Informationen der relativen Änderung der Durchflußmenge oder -geschwindigkeit sowie räumlichen Lage des Blutflusses und/oder intra- und extrakorporal fließender Flüssigkeiten im Gewebe gewinnt.

Dieses Auswerteverfahren nutzt die Eigenschaft der Wechselwirkung von elektromagnetischen Wellen an Gewebe und Blut- und/oder Flüssigkeitsbestandteilen aus. Aufgrund einer ausreichend kleinen Wellenlänge und dem damit verbunden, hinreichenden Verhältnis von Wellenlänge zu geometrischer Abmessung des zu detektierenden Körpers bzw. Gewebes, eignet sich der Spektralbereich von sichtbaren bis infraroten elektromagnetischen Wellen. Gleichzeitig wird eine ausreichende Detektionstiefe in biologischem Gewebe erreicht. Dieses Verfahren ist nicht invasiv und die aus den Wechselwirkungen entstehenden Änderungen sind proportional zur Geschwindigkeit, der Anzahl und der Tiefe der einzelnen sich bewegenden Blut und/oder Flüssigkeitsbestandteilen, so daß aus diesen Informationen eindeutig, insbesondere eine relative Änderung der Durchflußmenge o. dgl. nachweisbar und zuweisbar ist. Untersuchungen sowohl im klinischen Alltag als auch bei speziellen, medizinischen, pharmakologischen oder industriellen Fragestellungen zur tiefenselektiven Detektion des Blutflusses und/oder intra- und/oder extrakorporal fließender Flüssigkeiten lassen sich mit dieser Vorrichtung bzw. diesem Auswerteverfahren äußerst einfach, schmerzfrei und reproduzierbar durchführen.

Die Erfindung basiert i. w. darauf, mittels Laserlicht o. dgl., kohärente und monochromatische elektromagnetische Strahlung auf die Hautoberfläche bzw. Gewebeoberfläche der zu untersuchenden Stellen einzustrahlen. Die Photonen dringen in das Gewebe ein und werden entsprechend der optischen Parameter des Gewebe gestreut bzw. absorbiert. Da die Streuung mit einer Änderung der Ausbreitungsrichtung der Photonen einhergeht, werden auch Photonen aus dem Gewebe remittiert, d.h. an die Oberfläche des Gewebe bzw. der Haut zurückgestreut und treten wieder aus dem Gewebe aus. Diese Remission der aus dem Gewebe wieder austretenden Photonen weist eine abnehmende Intensität bei zunehmenden Abstand von dem Eintrittsort der Photonen auf. Ein weiteres Merkmal des biologischen Gewebes ist es, daß das Licht nicht gleichmäßig, d.h. isotrop, in alle Richtungen gestreut wird, sondern eine Vorwärtscharakteristik beim Streuprozeß erhalten bleibt. Das drückt sich in dem sogenannten Anisotropiefaktor g für Streuprozesse aus, der bei Gewebe einen Wert g ungefähr 0,9 annimmt. Ein Wert g = 0 würde isotroper, ein Wert g = 1 reiner Vorwärtsstreuung entsprechen.

Anhand eines einfaches Modell soll im folgenden erläutert werden, inwieweit über eine Detektion der remittierten Photonen ein Rückschluß auf den Zustand des Gewebes bzw. des Blutflusses und/oder intra- und/oder extrakorporal fließender Flüssigkeiten möglich ist: Betrachtet man z.B. Photonen, die etwa 5 mm neben dem Einstrahlort der Photonen aus dem Gewebe wieder heraustreten, dann kann mit hoher Wahrscheinlichkeit davon ausgegangen werden, daß sich diese wieder austretenden Photonen durch verschiedene Streuprozesse in etwa auf einer halbkreisförmigen oder ähnlichen Bahnkurve durch das Gewebe bewegt haben. Aufgrund der speziellen Durchführung des Auswerteverfahrens ist jedoch sicher, daß der Beginn der Bahnkurve am Eintrittsort der Photonen und das Ende der Bahnkurve am Meßpunkt der austretenden Photonen liegt. Sofern diese wieder austretenden Photonen überhaupt eine Information bzgl. der Bewegung des Blutflusses und/oder intra- und/oder extrakorporal fließender Flüssigkeiten haben sollten, kann jedenfalls davon ausgegangen werden, daß die direkt neben dem Einstrahlort wieder aus dem Gewebe austretenden Photonen nur Informationen bzgl. dicht unterhalb der Oberfläche durchströmter Gewebeschichten tragen, während solche in weiterem Abstand von dem Einstrahlort austretende Photonen Aufschluß auch über tiefer durchströmte Gewebeschichten geben können. Diese Modellbetrachtung verdeutlicht somit, daß durch eine Detektion von aus dem Gewebe wieder austretenden Photonen mit zunehmendem Abstand vom Einstrahlort selektiv Informationen aus bestimmten Gewebetiefen erhalten werden können.

Zur Messung bewegter, streuender Teilchen wird bekanntermaßen der optische Doppler-Effekt herangezogen. Dabei erfährt Licht beim Streuprozeß eine Frequenzverschiebung, die proportional zur Geschwindigkeit des bewegten Teilchens zunimmt. Unter Berücksichtigung des Doppler-Effektes kann so z.B. in oberflächlichen Gewebeschichten der Blutfluß im Gewebe bestimmt werden. Um ein optimales Doppler-Signal des Blutflusses und/oder intra- und/oder extrakorporal fließender Flüssigkeiten in tieferen Gewebeschichten zu erhalten, ist eine Lichtwellenlänge der Lichtquelle, insbesondere des Lasers, erforderlich, die vom Gewebe nur wenig absorbiert wird. Es bieten sich daher Wellenlängen im Bereich von etwa 600 nm bis etwa 1200 nm, bevorzugt bei etwa 820 nm an.

In der Literatur wird hin und wieder diskutiert, daß kohärentes Laserlicht beim Einstrahlen in das Gewebe durch die Vielzahl der Streuprozesse seine Kohärenzeigenschaften verlieren könnte. Durch interferometrische Untersuchungen läßt sich jedoch zeigen, daß ein gewisser Anteil von Photonen, die im größeren Abstand vom Einstrahlort aus dem Gewebe austreten, mit dem einfallenden Photonenstrahl interferieren kann, woraus zweifellos gefolgert werden muß, daß die Kohärenzeigenschaften dieser gestreuten Photonen noch vorhanden sind. Somit ist es aber auch möglich, in größeren Abständen der Detektoren von dem Einstrahlort noch Doppler-Signale mit der entsprechenden Frequenzverschiebung der wieder austretenden, frequenzverschobenen Photonen in/aus verschiedenen Tiefen zu detektieren. Dabei mischen sich die Photonen mit Dopplerverschobener Frequenz mit solchen Photonen, die keine Doppler-Verschiebung erfahren haben, also nur von einer starren bzw. unbeweglichen Matrix gestreut wurden. Am Detektionsort an der Gewebeoberfläche entsteht somit eine Intensitätsschwebung zwischen frequenzverschobenen und nicht-frequenzverschobenen Photonen. Dies führt zu einem lokalen Speckle-Muster, dessen Intensität mit der Doppler-Frequenz variiert und somit von einem optischen Detektor gemessen werden kann.

Im Prinzip erfolgt die Auswertung der Signale in der Form, daß zwei Detektoren, bevorzugt in fortlaufenden Abständen zur Einstrahlphase jeweils symmetrisch zum Einstrahlort die remittierten Photonen erfassen. Die Ausgangssignale dieser Detektoren werden entsprechend ausgewertet und verarbeitet, um zu den gewünschten Informationen bzw. Aussagen hinsichtlich des Blutflusses und/oder intra- und/oder extrakorporal fließender Flüssigkeiten zu gelangen.

Zusammenfassend kann festgestellt werden, daß ein derartiges Auswerteverfahren besonders im Bereich des "therapeutischen Fensters", also bei Wellenlängen im Bereich von 600 nm bis 1200 nm, in dem die Streuung der eingestrahlten Photonen nicht vernachlässigbar ist, durchführbar ist. Die optische Absorption aus Streuung von Licht in menschlichem Gewebe kann durch die Photonentransporttheorie näher beschrieben werden. Hierbei wird der Pfad eines in die Haut eingestreuten Photons verfolgt. Das Photon erfährt an den einzelnen lokalen Streuern entweder eine elastische Streuung oder es wird vollständig absorbiert. Daraus läßt sich für Laserlicht des roten Wellenlängenbereichs (600 nm) bzw. des infraroten Wellenlängenbereichs (1200 nm) die Eindringtiefe und der Streuprozeß bestimmen. Obwohl die sogenannte mittlere freie Weglänge relativ kurz ist, kann Licht dieses Wellenlängenbereichs tief in das Gewebe eindringen, da die Streuung hauptsächlich in Vorwärtsrichtung erfolgt (sogenannte Mie-Streuung), die Streuvorgänge wesentlich häufiger sind als die der Absorption und die Absorption im Gewebe für diesen Wellenlängenbereich gering ist gegenüber anderen Wellenlängen. Die Lichtausbreitung im Gewebe wird nach der Transporttheorie durch folgende Parameter beschrieben:
Anisotropiefaktor, Streukoeffizient, Absorptionskoeffizient, mittlere freie Weglänge.

Eine Gesamtschau der durchgeführten theoretischen wie auch experimentellen Untersuchungen deutet darauf hin, daß mit dem erfindungsgemäßen Verfahren ringförmige Interferenzstrukturen in konzentrischer Lage bzgl. des Einstrahlortes erhältlich sind. Mit zunehmendem lateralen Abstand zum Einstrahlpunkt legen die Photonen mit hoher Wahrscheinlichkeit im Gewebe größere Wege zurück und dringen dementsprechend auch tiefer in das Gewebe ein. Um die im Photonenzustand enthaltene Information über Bewegungen des ausgeleuchteten Gewebes auswerten zu können, sollte die Lichtquelle spezifische Eigenschaften, wie eine ausreichende Kohärenzlänge besitzen, monochromatisch und im single-Mode-Zustand betreibbar sein.

Eine wichtige Voraussetzung zum Erhalt der gewünschten Information ist eine ausreichend hohe Kohärenzlänge, so daß ein Interferenzmuster erzielbar ist. Das Entstehen des Interferenzmusters ist auf die Annahme zurückzuführen, daß Photonen, die nahe der Gewebe- bzw. Hautoberfläche gestreut werden, keine Frequenzveränderungen erfahren, wobei diese sozusagen nicht frequenzverschobene Photonen mit solchen Photonen, die in der Tiefe an bewegten Teilchen, also an den sich bewegenden Blut- und/oder Flüssigkeitsbestandteilen gestreut werden und dadurch eine Frequenzveränderung erfahren, interferieren. Wird daher das frequenzverschobene Streulicht, welches an sich bewegenden Teilchen gestreut wurde, mit frequenzunverschobenem quasi Originallicht, auf der Detektorfläche zur Deckung gebracht, entsteht eine Schwebungsfrequenz bzw. ein Interferenzmuster. Um diese Schwebungsfrequenzen aus tieferen Gewebeschichten zu erhalten, ist der Einsatz einer Wellenlänge im Bereich von 600 nm bis ca. 1200 nm erforderlich, wobei ebenfalls auf eine ausreichende Kohärenzlänge der Lichtquelle geachtet werden sollte. Es konnte gezeigt werden, daß typische Interferenzmuster auch für große Gewebetiefen nachweisbar sind und daß die Informationen mit zunehmendem Abstand der Detektorfläche aus zunehmender Tiefe des Gewebes stammt.

Dieser Nachweis ist auch für große laterale Abstände der Detektorfläche vom Einstrahlort in einem Bereich von bis zu ca. 15 mm erfolgreich durchgeführt worden. Allerdings erscheinen auch laterale Abstände bis 30 mm in der Praxis grundsätzlich möglich zu sein.

Eine besonders vorteilhafte Ausgestaltung des eingangs geschilderten, erfindungsgemäßen Auswerteverfahrens besteht darin, daß man eine Lichtquelle mit großer Kohärenzlänge, insbesondere größer als 10 cm, verwendet und man die wieder austretenden Photonen jeweils in einem bestimmten Abstand von dem ersten Bereich, insbesondere zeitgleich wenigstens in zwei dicht benachbarten oder symmetrisch zum ersten Bereich angeordneten Flächenbereichen, detektiert. Durch diese Maßnahme wird die Voraussetzung für ein besonders gutes Signal-/Rausch-Verhältnis geschaffen.

Dabei hat es sich als vorteilhaft erwiesen, daß man die detektierten Signale jeweils zweier Flächenbereiche unter anderem einer Differenzverstärkerfunktion zuführt sowie ggf. einer adaptiven Filterfunktion und ggf. einer Frequenzund/oder Cepstrumanalyse unterwirft. Durch diese Art der verfahrensmäßigen Signalauswertung kann eine genaue Analyse der Muskelaktivitäten erhalten werden.

Weitere Vorteile und Anwendungsmöglichkeiten der vorliegenden Erfindung ergeben sich aus der nachfolgenden Beschreibung der Ausführungsbeispiele anhand der Zeichnungen.

Es zeigen:
- **Figur 1**: eine schematische Ansicht eines ersten Ausführungsbeispiels der erfindungsgemäßen, auf ein Gewebe aufgesetzten Vorrichtung in Seitenansicht,
- Figur 2: ein zweites Ausführungsbeispiel der erfindungsgemäßen Vorrichtung in Seiten- und Unteransicht,
- Figur 3: ein drittes Ausführungsbeispiel der erfindungsgemäßen Vorrichtung in schematischer Darstellung,
- Figur 4: ein Blockdiagramm einer adaptiven Filterfunktion und
- Figur 5: ein Blockschaltbild einer Cepstrumanalysefunktion.

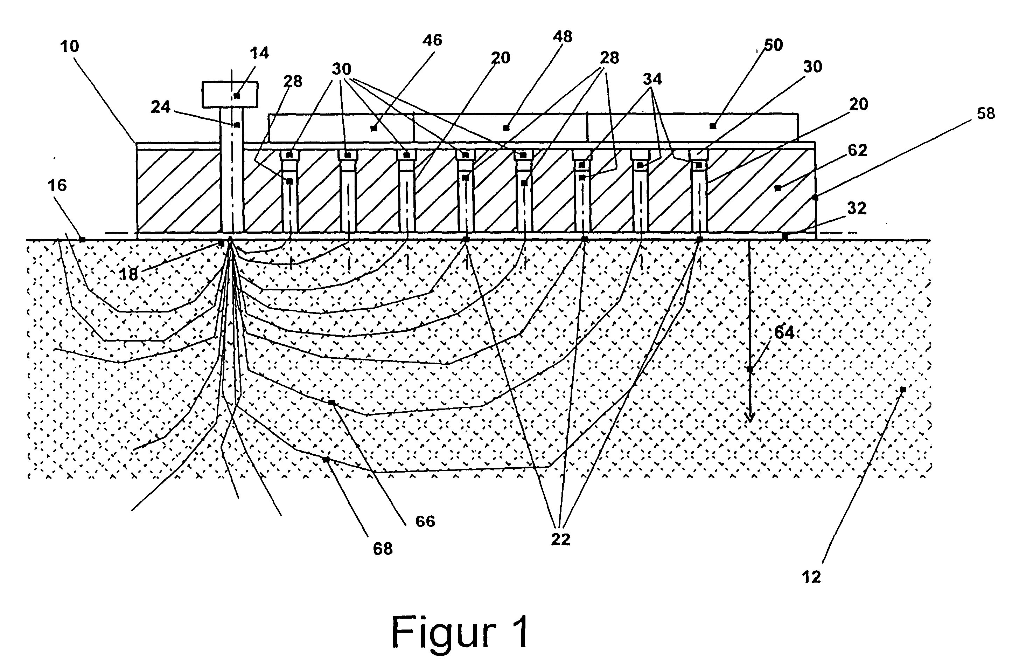

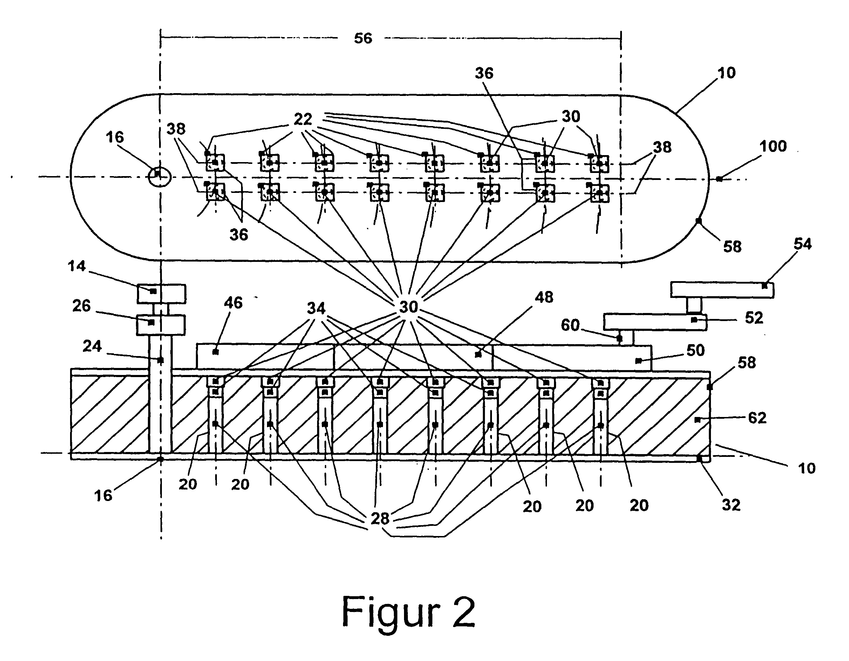

Die in den Figuren 1 und 2 dargestellte Vorrichtung 10 zur tiefenselektiven Detektion des Blutflusses und/oder intraund/oder extrakorporal fließender Flüssigkeiten in menschlichem, tierischem o. dgl. Gewebe 12 weist eine Lichtquelle 14, insbesondere einen Halbleiterlaser, zur Aussendung von Photonen hinein in das Gewebe 12 bzw. die Blutgefäße auf. Die Vorrichtung 10 ist mit einer Trägerplatte auf das Gewebe 12 bzw. die Haut 18 des Gewebes aufgesetzt, wobei Photonen durch einen ersten Bereich 16, der örtlich i. w. wohl definiert ist, in das Gewebe 12 bzw. die Blutgefäße eintreten. Die Photonen werden in dem Gewebe 12 bzw. den Blutgefäßen zum Teil gestreut, zum Teil absorbiert, wobei einige mögliche Photonenwegstrecken 66, 68 bildlich und schematisch in Figur 1 dargestellt sind. Deutlich ist sichtbar, daß die Photonen mit wachsender Eindringtiefe 64 immer weiter entfernt von dem ersten Bereich 16 aus dem Gewebe 12 wieder austreten.

Weiterhin weist die Vorrichtung 10 mehrere Detektoren 20 zur Erfassung der aus weiteren Flächenbereichen 22 der Haut 18 bzw. des Gewebes 12 austretenden Photonen auf. Die weiteren Flächenbereiche 22 sind in unterschiedlichen Abständen von dem ersten Bereich 16 beabstandet.

Der Lichtquelle 14 ist gemäß dem Ausführungsbeispiel der Figur 1 ein dem Gewebe 12 stirnseitiges aufsetzbares Lichtleitfaserstück 24 zugeordnet. Aus dem Ausführungsbeispiel der Figur 2 ist ersichtlich, daß jedoch anstelle des Leichtleitfaserstücks 24 auch eine Kollimationsoptik 26 zur Fokussierung des Laserlichts auf dem Gewebe 12 bzw. der Haut 18 zum Einsatz kommen kann.

Die Detektoren 20 weisen jeweils eine dem Gewebe 12 stirnseitig aufsetzbare Lichtleitfaser 28 auf, denen eine Photodiode 30 o. dgl. nachgeordnet ist.

Der Lichtquelle 14 ist ein Polarisationsfilter nach- und den Detektoren 20 vorgeschaltet. Weiterhin sind zwischen die Lichtleitfaser 28 und die Photodiode 30 ggf. Absorptionsfilter 34 eingebracht.

Die Wellenlänge des Halbleiterlasers liegt im Bereich von etwa 600 nm bis etwa 1200 nm, bevorzugt bei etwa 820 nm.

Wie insbesondere aus dem Ausführungsbeispiel der Figur 2 ersichtlich ist, sind die weiteren Flächenbereiche 22 bzw. die zugeordneten Detektoren 20 paarweise einander benachbart und i. w. in Reihe 38 liegend hintereinander angeordnet, wobei jedes Paar 36 einen anderen Abstand zum ersten Bereich 16 aufweist und die Abstände benachbarter Paare 36 jedenfalls im Ausführungsbeispiel äquidistant sind. Es versteht sich, daß auch andere Abstände der einzelnen Detektoren 20 bzgl. des ersten Bereichs 16 gewählt werden können, dies bemißt sich anhand der speziellen Erfordernisse des jeweiligen Systems und anhand des Fachwissens des Durchschnittsfachmanns. Die weiteren Flächenbereiche 22 bzw. die entsprechenden Detektoren 20 sind bis zu einem maximalen Abstand 56 von etwa 15 bis 30 mm von dem ersten Bereich 16 angeordnet.

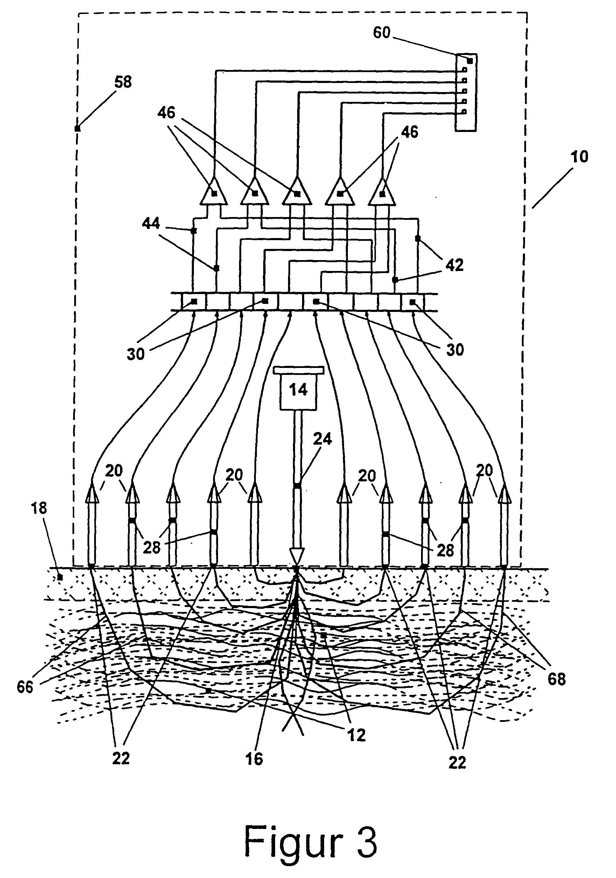

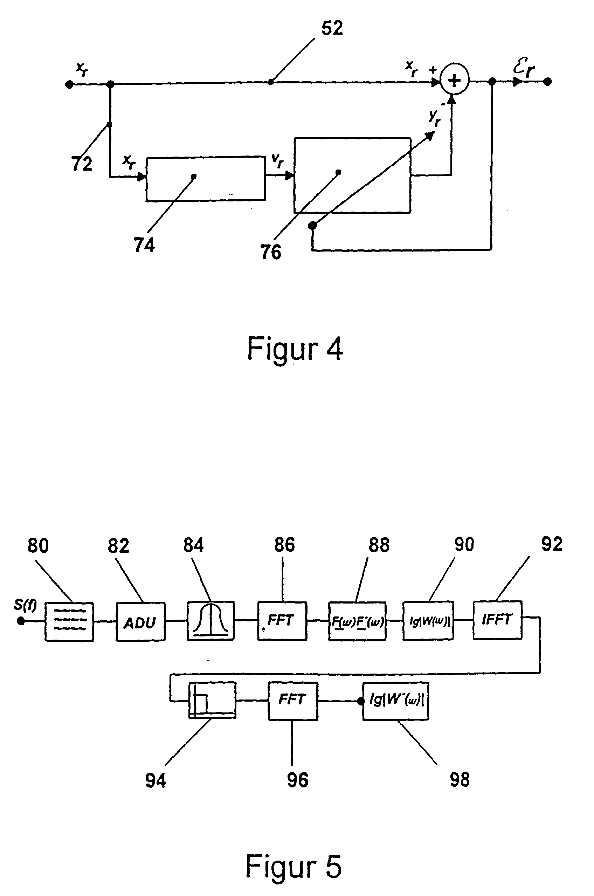

Wie insbesondere aus Figur 3 ersichtlich ist, werden die von paarweisen Flächenbereichen 22 bzw. von den entsprechenden Detektoren 20 erfaßten beiden Signale 42, 44 jeweils den beiden Eingängen eines Differenzverstärkers 46 zugeführt. Diese Maßnahme findet vorteilhafterweise auch von Vorteil bei den Ausführungsbeispielen der Figuren 1 und 2 Anwendung, wobei dann entsprechende Paare 36 von Detektoren an einen Differenzverstärker 46 angeschlossen sind. Die Signale der Detektoren 20 werden unter anderem einer adaptiven Filterfunktion 52 sowie einer Cepstrumanalysefunktion 78 unterworfen (Figur 5).

Die gesamte Vorrichtung mit Ausnahme eines Prozessors 54, also die Lichtquelle 14, die Detektoren 20 und die elektronischen Komponenten, wie ggf. Vorverstärker 48, Differenzverstärker 46 sowie Analog/Digital-Wandler 50 sind gemeinsam in einem Meßkopf 58 untergebracht, der flächig auf das Gewebe 12 bzw. die Haut 18 auflegbar ist. Der Meßkopf 58 weist somit lediglich eine Verbindung mittels elektrischer Leiter zu der Auswerteeinheit, insbesondere dem Prozessor 54 auf. Das Innere des Meßkopfes ist mit einer Füllmasse 62 ausgefüllt.

In Abweichung zu der Anordnung der Detektoren 20 der Ausführungsformen der Figur 1 und 2 weist die Ausführungsform gemäß der Figur 3 weitere Flächenbereiche 22 bzw. zugeordnete Detektoren 20 auf, die i. w. auf einer durch den ersten Bereich 16 laufenden Geraden 40 und paarweise symmetrisch beidseitig des ersten Bereichs 16 angeordnet sind.

Die adaptive Filterfunktion 52 in Figur 4 weist zwei Kanäle 70, 72 auf, wobei der erste Kanal 70 das unveränderte Eingangssignal führt, in dem Kanal 72 jedoch eine Verzögerungsstufe 74 eingeschaltet ist. Mittels der Adaptionsstufe 76 werden die Filterkoeffizienten solange geändert, bis die Differenz zwischen dem ungefilterten Eingangssignal des Kanals 70 und des gefilterten Signals des Kanals 72, im quadratischen Mittel minimal wird.

Gemäß Figur 5, welche ein Blockschaltbild der Cepstrumanalysefunktion 78 wiedergibt, werden die Signale als Zeit-Amplituden-Funktion graphisch dargestellt und mit einer reellwertigen Fast-Hartley-Transformation (FHT) bzw. einer Fast-Fourier-Transformation (FFT) oder einem anderen Frequenzanalysealgorithmus in ein Leistungsspektrum 88 bzw. Powerspektrum zerlegt. Hierbei werden die in der Schwebungsfrequenz enthaltenen Teilfrequenzen hinsichtlich ihrer Intensität und Bandbreite analysiert. Das Leistungsspektrum 88 wird in Echtzeit mit einer Stützpunktzahl größer 64 Punkte ermittelt und graphisch für jeden A/D-Wandler-Kanal 82 in eine Art "Wasserfalldarstellung" aufbereitet. Dieses Leistungsspektrum wird einer Momentenanalyse sowohl für die Intensität als auch für die Frequenz unterworfen. Die so gebildeten Momente lassen sich zu einem Vektor zusammenfassen und graphisch darstellen. Insgesamt besteht die Cepstrumanalysefunktion 78 aus der Hintereinanderschaltung einer Filterfunktion 80, eines A-/D-Umsetzers 82, eines diskreten Zeitfensters 84, einer Fast-Fourier-Transformation 86, einem Leistungsspektrum 88, einer Logarithmierfunktion 90, einer inversen Fast-Fourier-Transformation 92, einer Lifterfunktion 94, einer Fast-Fourier-Transformation 96 und schließlich dem modifizierten Leistungsspektrum 89. Dieser letztere Vorgang wird auch als Cepstrumanalysefunktion 78 bezeichnet, wodurch charakteristische, sich verändernde Frequenzen sichtbar gemacht werden können. Die Bedeutung der Cepstrumanalysefunktion liegt insbesondere darin, daß Spektren mit periodischen Schwankungen einer genauen Analyse unterzogen werden können. Das Cepstrum wird aus dem phasenlosen Leistungsspektrum gebildet.

Alle Meßdaten, Auswertungen und Analysen können auf einer graphischen Benutzeroberfläche dem Anwender visualisiert und zur weiteren Bearbeitung auf Massenspeichern archiviert werden. Die verschiedenen Signale der einzelnen Kanäle können auch weiterführend mit einer Kreuzkorrelation, der Autokorrelation und anderen Signalauswertealgorithmen miteinander verglichen und auswertet werden.

Die Ausführungsform der Figuren 1 und 2 funktioniert i. w. wie folgt. Ein in dem Meßkopf 58 angeordneter Halbleiterlaser strahlt kohärentes, monochromatisches Licht, insbesondere einer Kohärenzlänge größer als 10 cm, direkt in das Lichtleitfaserstück 24 ein. Das Licht wird in dem Lichtleitfaserstück 24 zur Oberfläche der Haut 18 in dem ersten Bereich 16 weitergeleitet. Das Lichtleitfaserstück 24 sollte vorzugsweise eine Mono-Mode-Faser mit einem Mindestdurchmesser von ca. 300 µm sein, um die Lichtleistung des Halbleiterlasers übertragen zu können. Je nach Ausführung kann auf das Lichtleitfaserstück 24 verzichtet werden, wenn das Laserlicht mit Hilfe einer Kollimationsoptik 26 unmittelbar auf die Hautoberfläche fokussiert wird. Das remittierte Licht wird an den Lichtleitfasern 28 in den weiteren Flächenbereichen 22 aufgenommen und zu den Photodioden 30 weitergeleitet. Vor oder hinter den Lichtleitfasern 28 und/oder vor dem Lichtleitfaserstück 24 sitzen jeweils Absorptionsfilter 34 bzw. Polariationsfilter 32.

Alle Fasern und Filter sind bevorzugt in einer lichtundurchlässigen Kunststoff- oder Keramikfassung auf dem Meßkopf 58 arretiert. Die Fasern sind paarweise außermittig einer Längsachse 100 auf einer Trägerplatte des Meßkopfes 58 angeordnet. Der Abstand zwischen den einzelnen Paaren 36 ist gleich, wobei dieser Abstand in verschiedenen Ausführungen variabel gestaltet werden kann. Die Lichtleitfasern 28 sollten einen Durchmesser von 400 µm nicht überschreiten, da das Signal-/Rauschverhältnis mit kleiner werdendem Durchmesser besser wird. Allerdings nimmt auch mit kleiner werdendem Durchmesser die Intensität des Meßsignals ab, so daß hier ein Kompromiß zu finden ist. Bei der Wahl der Photodioden 30 ist bevorzugt darauf zu achten, daß die einzelnen Photoströme bei gleicher Ausleuchtung nur minimal voneinander abweichen. Die gesamte Auswerteelektronik bestehend aus Vorverstärker 48 und Differenzverstärker 46 ist in dem abgeschirmten Gehäuse des Meßkopfs 58 integriert. Auch die Analog-/Digital-Wandler 50 können im Meßkopf 58 angeordnet sein. Allerdings besteht auch die Möglichkeit, diese Komponenten außerhalb des Meßkopfes 58 auf einer Meßkarte zu plazieren, so daß nur digitalisierte Signale an den Prozessor 54 weitergegeben werden. Die Auswerteelektronik hat die Aufgabe, den von den Photodioden 30 kommenden Strom in eine Spannung zu wandeln und zu verstärken, was mittels des Vorverstärkers 48 durchgeführt wird. Anschließend wird von paarweise angeordneten und geschalteten Photodioden 30 mittels der Differenzverstärker 46 eine Signaldifferenz gebildet und weiter verstärkt. Schließlich ist jedem Differenzverstärker 46 ein Analog-/Digital-Wandler 50 mit 12 bis 16 bit Auflösung und einer Mindestabtastrate von etwa 20 kHz nachgeschaltet. Somit wird jedem Paar 36 der Photodioden ein A/D-Wandler-Kanal zugeordnet. Die digitalisierten Signale werden zur Auswertung dem Prozessor 54 zugeleitet.

Es bleibt noch zu erwähnen, daß im Unterschied zur Ausführung der Figuren 1 und 2 im Ausführungsbeispiel gemäß Figur 3 die Paare 36 der Photodioden 30 bzw. der Flächenbereiche 22 nicht paarweise nebeneinander angeordnet sind, sondern sozusagen diametral und symmetrisch links bzw. rechts des ersten Bereichs 16 ausgebildet sind. Auch bei dieser Anordnung lassen sich mittels einer Differenzbildung der Ausgangssignale entsprechender Paare 36 von Photodioden 30 verbesserte Signal-/Rauschverhältnisse bilden. Allerdings kann diese Anordnung, insbesondere bei Vorhandensein einer Vorzugsrichtung der Lichtausbreitung in dem Gewebe, von gewissem Nachteil sein.

Insoweit handelt es sich bei den dargestellten Ausführungsformen der Figur 1 und 2 bzgl. der paarigen Anordnung der Photodioden 30 bzw. weiteren Flächenbereiche 22 um eine bevorzugte Ausführungsform der Erfindung.

### Bezugszeichenliste

- 10 -: Vorrichtung
- 12 -: Gewebe
- 14 -: Lichtquelle
- 16 -: erster Bereich
- 18 -: Haut
- 20 -: Detektor
- 22 -: Flächenbereich
- 24 -: Lichtleitfaserstück
- 26 -: Kollimationsoptik
- 28 -: Lichtleitfaser
- 30 -: Photodiode
- 32 -: Polariationsfilter
- 34 -: Absorptionsfilter
- 36 -: Paar
- 38 -: Reihe
- 40 -: Gerade
- 42 -: Signal
- 44 -: Signal
- 46 -: Differenzverstärker
- 48 -: Vorverstärker
- 50 -: A/D-Wandler
- 52 -: Filterfunktion
- 54 -: Prozessor
- 56 -: Abstand
- 58 -: Meßkopf
- 60 -: Leiter
- 62 -: Füllmasse
- 64 -: Eindringtiefe
- 66 -: Photonenwegstrecke
- 68 -: Photonenwegstrecke
- 70 -: Kanal
- 72 -: Kanal
- 74 -: Verzögerungsstufe
- 76 -: Adaptionsstufe
- 78 -: Cepstrumanalysefunktion
- 80 -: Filterfunktion
- 82 -: A/D-Wandler
- 84 -: Zeitfenster
- 86 -: Fast-Fourier-Transformation
- 88 -: Leistungsspektrum
- 90 -: Logarithmierfunktion
- 92 -: inverse Fast-Fourier-Transformation
- 94 -: Lifterfunktion
- 96 -: Fast-Fourier-Transformation
- 98 -: mod. Leistungsspektrum
- 100 -: Längsachse

## Patentansprüche

1. Auswerteverfahren zur Detektion des Blutflusses und/oder intra- und/oder extrakorporal fließender Flüssigkeiten in menschlichem, tierischem oder biologischem Gewebe (12), wobei man Photonen einer kohärenten, monochromatischen Lichtquelle (14) in das Gewebe (12) durch einen ersten Bereich (16) eintreten läßt, man in beabstandeten Flächenbereichen (22) aus dem Gewebe wieder austretende Photonen bezüglich Frequenz und Anzahl beziehungsweise Intensität detektiert, **dadurch gekennzeichnet, daß** die Flächenbereiche (22) in unterschiedlichen Abständen von dem ersten Bereich (16) angeordnet sind und man aus detektierten Interferenzmustern oder Frequenzschwebungen in Verbindung mit dem jeweiligen Flächenbereich (22) als Austrittsort der wieder austretenden Photonen mittels eines Auswerteprogramms oder -algorithmus tiefenselektive Informationen der relativen Änderung der Durchflußmenge oder -geschwindigkeit sowie räumlichen Lage des Blutflusses und/oder intra- und extrakorporal fließender Flüssigkeiten im Gewebe (12) gewinnt.

2. Auswerteverfahren nach Anspruch 1, **dadurch gekennzeichnet, daß** man eine Lichtquelle (14) mit einer großen Kohärenzlänge, insbesondere größer als 10 cm, verwendet, und man die jeweils in einem bestimmten Abstand von dem ersten Bereich (16) wieder austretenden Photonen, insbesondere zeitgleich wenigstens in zwei dicht benachbarten oder symmetrisch zum ersten Bereich (16) angeordneten Flächenbereichen (22) detektiert.

3. Auswerteverfahren nach Anspruch 2, **dadurch gekennzeichnet, daß** man die detektierten Signale jeweils zweier Flächenbereiche (22) unter anderem einer Differenzverstärkerfunktion (46) zuführt, sowie ggf. einer adaptiven Filterfunktion (52) und ggf. einer Frequenzund/oder Cepstrumanalysefunktion (78) unterwirft.

## Claims

1. Evaluating method for detection of blood flow and/or intracorporally and/or extracorporally flowing liquids in human, animal or biological tissue (12), wherein photons of a coherent monochromatic light source (14) are allowed to enter the tissue (12) through a first region (16) and photons exiting the tissue again are detected in spaced surface regions (22) with respect to frequency and number or intensity, **characterised in that** the surface regions (22) are arranged at different spacings from the first region (16) and depth-selective data concerning the relative change in throughflow quantity or throughflow speed and physical position of the blood flow and/or intracorporally and/or extracorporally flowing liquids in the tissue (12) are obtained by means of an evaluating program or evaluating algorithm from detected interference patterns or frequency fluctuations in conjunction with the respective surface region (22) as exit location of the photons exiting again.

2. Evaluating method according to claim 1, **characterised in that** a light source (14) with a large coherence length, in particular greater than 10 cm, is used and the photons exiting again in each instance at a specific spacing from the first region (16) are detected, in particular isochronously, in two surface regions (22) which are closely adjacent or arranged symmetrically relative to the first region (16).

3. Evaluating method according to claim 2, **characterised in that** the detected signals in each instance of two surface regions (22) are fed inter alia to a differential amplifier function (46) as well as subjected optionally to an adaptive filter function (52) and optionally to a frequency and/or cepstrum analysis function (78).

## Revendications

1. Procédé de test pour la détection du flux sanguin et/ou de liquides intra et/ou extra corporels circulant dans un tissu humain, animal ou biologique (12), dans lequel on fait entrer des photons d'une source de lumière cohérente monochromatique (14) dans le tissu (12) à travers une première région (16), on détecte, sous l'aspect de la fréquence et du nombre, respectivement de l'intensité, les photons qui ressortent du tissu dans des régions éloignées (22) de la surface, **caractérisé en ce que** les régions (22) de la surface sont disposées à des distances différentes par rapport à la première région (16) et qu'on obtient, à partir de figures d'interférence détectées ou de fluctuations de la fréquence en relation avec chacune des régions (22) de la surface qui constitue le lieu de sortie des photons qui ressortent, et au moyen d'un programme ou algorithme de test, des informations sélectives en fonction de la profondeur, sur la variation relative du débit ou de la vitesse d'écoulement, ainsi que sur la position dans l'espace du flux de sang et/ou des liquides situés dans le tissu (12) et qui sont en circulation intra-corporelle et extra-corporelle.

2. Procédé de test selon la revendication 1, **caractérisé en ce qu'**on utilise une source de lumière (14) ayant une grande longueur de cohérence, en particulier supérieure à 10 cm, et on détecte les photons qui ressortent à une distance donnée de la première région (16), en particulier en isochronisme au moins dans deux régions de surface (22) étroitement adjacentes ou disposées symétriquement par rapport à la première région (16).

3. Procédé de test selon la revendication 2, **caractérisé en ce qu'**on achemine les signaux détectés de deux régions de surface (22), entre autres, vers une fonction d'amplificateur différentiel (46) et qu'on les soumet éventuellement à une fonction filtre adaptative (52) et éventuellement à une fonction d'analyse de la fréquence et/ou du cepstre (78).
